# EUROPEAN PATENT SPECIFICATION

(11) **EP 4 487 803 B1**
(45) Date of publication and mention of the grant of the patent: **22.10.2025**
(21) Application number: 23183372.4
(22) Date of filing: 04.07.2023
(51) Int. Cl.: A61B 34/20, A61B 34/00, A61B 34/30

(54) **TECHNIQUE FOR SUPPORTING CLINICAL PERSONNEL WITH MULTIPLE NAVIGATION VIEWS**
TECHNIK ZUR UNTERSTÜTZUNG VON KLINISCHEM PERSONAL MIT MEHREREN NAVIGATIONSANSICHTEN
TECHNIQUE D'ASSISTANCE DU PERSONNEL CLINIQUE AVEC DE MULTIPLES VUES DE NAVIGATION

(43) Date of publication of application: 08.01.2025
(73) Proprietor: Stryker European Operations Limited, Carrigtwohill, Co. Cork T45 HX08 (IE)
(72) Inventor: SCHÖPP, Hans, 79110 Freiburg (DE)
(74) Representative: Schott, Jakob Valentin

(56) References cited:
- EP-A1- 3 919 018
- WO-A1-2023/002382
- US-A1- 2019 228 859

## Description

### Technical Field

The present disclosure generally relates to a method for supporting clinical personnel with multiple navigation views. A system, a computer program and a carrier are also provided.

### Background

In many clinical scenarios, clinical personnel such as surgeons wish to be provided with feedback regarding tracked poses of medical instruments. For example, surgeons may wish to be informed on a current pose of a handheld drill relative to a patient's body, or on an alignment of a pedicle screw driver in relation to another medical instrument, such as a trocar handled by a robot.

Some surgical navigation systems can provide a surgeon with a single navigation view visualizing a tracked pose of a handheld medical instrument relative to a patient's body as represented by patient image data. In case multiple medical instruments are used, tracked poses of the multiple medical instruments may be visualized at the same time in the single navigation view. Especially if the tracked medical instruments are located far apart from one another (e.g., at different vertebral levels of a patient's spine), or if multiple medical instruments are handled simultaneously by different surgeons, a single navigation view may not be optimal for navigating each of the medical instruments.

US 2019/228859 A1 describes a surgical workflow system for performing a surgical procedure. The surgical workflow system comprises one or more locators arrangeable to determine locations of a plurality of surgical objects. Information conveyor devices are coupled to the one or more locators and are assigned to different participants in the surgical procedure. A workflow controller accesses a pre-scripted workflow having a plurality of workflow steps associated with the surgical procedure, identifies a change event that indicates a deviation from the pre-scripted workflow, determines event information tailored to the different participants based on the change event, and transmits the event information to the participants through the plurality of information conveyor devices so that different event information can be conveyed to the different participants, wherein the event information is provided in the context of the surgical objects.

WO 2023/002382 A1 describes systems, methods, and/or instrumentalities for a surgical hub configuring a display. In examples, a health care provider and/or a medical instrument may be tracked within an operating room. In examples, a first health care provider and a second health care provider may be tracked within an operating room. In examples, an health care provider and/or a patient may be tracked within an operating room. A surgical task that uses the medical instrument during a medical procedure may be determined.

EP 3 919 018 A1 discloses a technique of providing user guidance for surgical navigation. A method implementation of the technique comprises obtaining a predetermined spatial relationship between an optical tracking pattern and a through-hole extending through an implant, obtaining image data of the optical tracking pattern acquired by an imaging unit attached to a surgical instrument, obtaining a spatial relationship between the surgical instrument and the imaging unit at a point in time when the image data have been acquired, determining a spatial relationship between the surgical instrument and the through-hole, obtaining a plurality of predefined spatial relationships between the surgical instrument and the through-hole, and triggering simultaneous display of an indication of the plurality of predefined spatial relationships and an indication of the spatial relationship between the surgical instrument and the through-hole.

### Summary

There is a need for a technique for supporting clinical personnel that solves one or more of the aforementioned or other problems.

According to a first aspect, a method for supporting clinical personnel with multiple navigation views is provided. The method is performed by at least one processor and comprises obtaining patient tracking information indicative of tracked poses of two or more patient trackers, each of the patient trackers having a fixed spatial relationship relative to one or more anatomical elements of a patient's body. The method comprises obtaining instrument tracking information indicative of tracked poses of two or more medical instruments. The method comprises determining based on the patient tracking information and the instrument tracking information, multiple navigation views, wherein each of the navigation views is determined based on a respective set comprising (i) at least one of the medical instruments and (ii) at least one of the patient trackers, wherein the sets differ from one another in the at least one of the patient trackers. The method further comprises triggering simultaneous display of the multiple navigation views.

Each of the navigation views may be specific for the respective set used to determine the respective navigation view.

Each of the at least one of the patient trackers within a same set may have a fixed spatial relationship relative to a common anatomical element of the patient's body.

The common anatomical element may differ from one set to another.

The sets may differ from one another in the at least one of the medical instruments.

At least one of the sets may comprise at least one of the medical instruments that is not comprised in any other one of the sets. Alternatively, or in addition, at least one of the sets may comprise at least one of the medical instruments that is comprised in another one of the sets.

At least one of the sets may be determined based on the patient tracking information and the instrument tracking information.

At least one of the sets may be determined based on a relative pose between (i) the at least one of the medical instruments and (ii) at least one of the one or more anatomical elements having the fixed spatial relationship relative to the at least one of the patient trackers. The at least one of the sets may be determined based on the relative pose indicating that at least one of the following conditions is fulfilled: the at least one of the medical instruments is aligned with the at least one of the one or more anatomical elements having the fixed spatial relationship relative to the at least one of the patient trackers; the at least one of the medical instruments is positioned within a predefined region that is associated with the at least one of the one or more anatomical elements having the fixed spatial relationship relative to the at least one of the patient trackers.

At least one of the sets may be determined based on a user input.

At least one of the navigation views may indicate a relative pose between (i) a medical instrument of the set used to determine the at least one of the navigation views and (ii) at least one of the one or more anatomical elements having the fixed spatial relationship relative to one or more of the at least one of the patient trackers of the same set. The at least one of the navigation views may further indicate a pose of another medical instrument of the set or of another set.

Two or more of the navigation views may be triggered to be displayed on a common display unit. Alternatively, or in addition, at least one of the navigation views may be triggered to be displayed on an individual display unit.

At least one of the medical instruments may be handled by a robot. Alternatively, or in addition, at least two of the medical instruments may be handled (e.g. simultaneously) by different persons.

According to a second aspect, a system is provided. The system comprises at least one processor configured to: obtain patient tracking information indicative of tracked poses of two or more patient trackers, each of the patient trackers having a fixed spatial relationship relative to one or more anatomical elements of a patient's body; obtain instrument tracking information indicative of tracked poses of two or more medical instruments; and trigger, based on the patient tracking information and the instrument tracking information, simultaneous display of multiple navigation views, each of the navigation views being determined based on a respective set comprising (i) at least one of the medical instruments and (ii) at least one of the patient trackers, wherein the sets differ from one another in the at least one of the patient trackers. The at least one processor may be configured to perform the method according to the first aspect.

The system may further comprise one or more display units configured to simultaneously display the navigation views. The system may comprise a tracking unit configured to track at least one entity selected from the patient trackers and the medical instruments. The system may comprise a robot configured to handle at least one of the medical instruments.

According to a third aspect, a computer program is provided. The computer program comprises instructions which, when performed by at least one processor, configure the at least one processor to: obtain patient tracking information indicative of tracked poses of two or more patient trackers, each of the patient trackers having a fixed spatial relationship relative to one or more anatomical elements of a patient's body; obtain instrument tracking information indicative of tracked poses of two or more medical instruments; and trigger, based on the patient tracking information and the instrument tracking information, simultaneous display of multiple navigation views, each of the navigation views being determined based on a respective set comprising (i) at least one of the medical instruments and (ii) at least one of the patient trackers, wherein the sets differ from one another in the at least one of the patient trackers. The computer program may comprise instructions which, when performed by at least one processor, configure the at least one processor to perform the method according to the first aspect.

According to a third aspect, a carrier is provided. The carrier carries the computer program according to the third aspect. The carrier may be a data stream or a non-transitory computer-readable storage medium.

### Brief Description of the Drawings

Further details, advantages and aspects of the present disclosure will become apparent from the following embodiments taken in conjunction with the drawings, wherein:
- Fig. 1: shows a schematic illustration of a system in accordance with the present disclosure;
- Fig. 2: shows a flowchart of a method in accordance with the present disclosure; and
- Fig. 3: schematically illustrates exemplary navigation views in accordance with the present disclosure.

### Detailed Description

In the following description, exemplary embodiments will be explained with reference to the drawings. The same reference numerals will be used to denote the same or similar structural features.

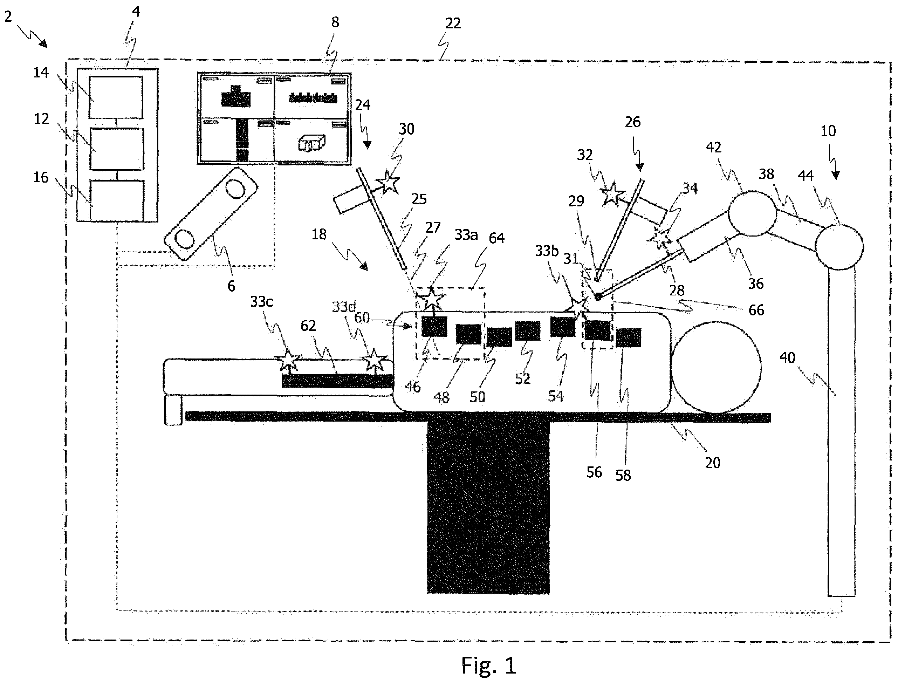

Fig. 1 shows a schematic illustration of a system 2 in accordance with the present disclosure. The system 2 may be referred to as a surgical navigation system and comprises a computing system 4. The system 2 may further comprise a tracking unit 6, a display unit 8 and/or a robot 10. The computing system 4 comprises at least one processor 12 communicatively connected to at least one memory 14 and at least one interface 16. The at least one interface 16 may be communicatively connected to the tracking unit 6, the display unit 8 and/or the robot 10 via one or more wired or wireless connections. The display unit 8 may be a standalone display screen, part of a surgical navigation station or part of a handheld or head-mounted display. The system 2 may comprise a plurality of display units 8 or one or more additional, different display units.

Also shown is a patient's body 18 of a patient lying on a treatment bed 20 in an operating room 22, and three medical instruments 24, 26, 28. Each of the medical instruments 24, 26 in this example is a handheld instrument such as a pointer, a drill, a chisel or a screwdriver. The medical instruments 24, 26 may be handled simultaneously, for example by different surgeons. The medical instrument 24 comprises a shaft 25 extending longitudinally along a shaft axis 27. The medical instrument 26 comprises a distal instrument tip 29. The medical instrument 28 in the illustrated example is (e.g., simultaneously) handled by the robot 10 and comprises a distal instrument tip 31.

The tracking unit 6 is configured to track poses of the medical instruments 24, 26 by locating (e.g., optical or electromagnetic) trackers 30, 32 attached to the medical instruments 24, 26. The tracking unit 6 may also track a pose of the medical instrument 28 by locating a tracker 34 attached thereto. The tracking unit 6 may be configured as an optical tracking unit and may comprise a (e.g., stereo-) tracking camera for locating optical trackers. The tracking unit 6 may alternatively be configured as an electromagnetic tracking unit and may comprise an electromagnetic field generator. Other types of tracking units are also possible.

Alternatively to tracking the medical instrument 28 with the tracking unit 6, a pose of the medical instrument 28 may be determined (e.g., by the at least one processor 12) based on a pose of the robot 10, for example based on rotations and/or translations of actuators of the robot 10. In the illustrated example, a pose of the medical instrument 28 may be determined based on angular orientations of arm segments 36, 38, 40 of the robot 10 defined by joints 42, 44 between the arm segments 36, 38, 40 of the robot 10.

The tracking unit 6 may be further configured to track a pose of different parts of the patient's body 18 by locating one or more patient trackers 33a-33cd each having a fixed spatial relationship relative to one or more anatomical elements of the patient's body 18 (e.g., adhesively attached to the patient's skin or clamped to an anatomical element of the patient's body). The medical instruments 24, 26, 28 and the patient trackers 33a-33d may be arranged in a same operating room 22 in which the patient's body 18 is located.

A registration between (e.g., pre-operative) patient image data of at least a portion of the patient's body 18 on the one hand and the patient's body 18 on the other hand may be used to transform (e.g., pre-planned) locations defined relative to the patient image data into (e.g., real-world) locations in the operating room 22. The registration may define a spatial relationship between a coordinate system of the patient image data and a real-world coordinate system defined by one or more of the patient trackers 33a-33d, for example in the form of a transformation matrix or transformation function. Various techniques for determining such patient-to-image registrations are known to those skilled in the art. For example, points acquired on a surface of the patient's body 18 using a tracked registration probe may be matched to a surface of the patient's body 18 as represented by the patient image data to obtain a registration.

The patient's body 18 comprises a plurality of anatomical elements such as organs or bones. In the illustrated example, vertebrae 46-58 of the patient's spine 60 are indicated as examples of anatomical elements of the patient's body 18. It is noted that although a patient's spine 60 typically comprises a total of 33 vertebrae, only seven vertebrae 46-58 are illustrated in Fig. 1 to provide a better overview. Also indicated in Fig. 1 is the femur 62 as another example of an anatomical element of the patient's body 18, with two patient trackers 33c, 33d having a fixed spatial relationship relative to the femur 62. Fig. 1 further illustrates a spatial regions 64, 66. The spatial region 64 is associated with the tracker 33a and encloses the vertebrae 46, 48. The spatial region 66 is associated with the tracker 33b and contains the vertebra 56. Each spatial region may be defined by a user or automatically, for example as a region defining a predefined space around the enclosed anatomical element.

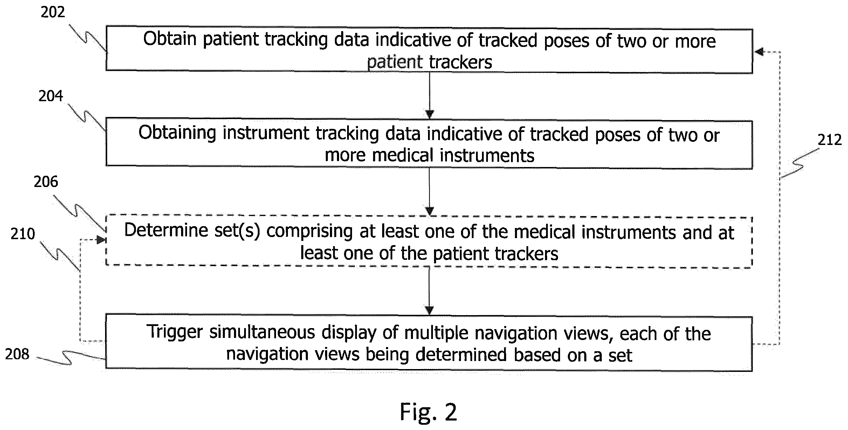

Fig. 2 shows a flowchart of a method in accordance with the present disclosure. The method may be performed by the at least one processor 12 of the system 2. To this end, the at least one memory 14 may store a computer program comprising instructions which, when executed by the at least one processor 12, cause the at least one processor to perform the method disclosed herein. The at least one memory 14 is an example of a carrier carrying the computer program. The computer program may alternatively be carried by a data stream received by the at least one processor 12 via the interface 16, for example from a server. Although reference signs of Fig. 1 will be used in the following to provide a better understanding of the method, these are not to be understood as limiting the method to the specific details shown in Fig. 1.

The method is a computer-implemented method. According to the invention, the method is not a method for treatment of the human or animal body by surgery or therapy. The method does not comprise a surgical step.

In step 202, patient tracking information indicative of tracked poses of two or more patient trackers 33a-33d is obtained.

The patient tracking information may be obtained from the tracking unit 6 and may indicate a (e.g., current) tracked pose for each of the two or more patient trackers 33a-33d.

Each of the patient trackers 33a-33d has a fixed spatial relationship relative to one or more anatomical elements 46-62 of the patient's body 18.

An anatomical element may be understood as a physically and/or biologically distinguishable part of the patient's body 18. Examples of anatomical elements include bones (e.g., vertebrae) 46-62, organs and tumors. In one example, each of the anatomical elements may not deform (e.g., not more than a predefined amount, e.g., no more than 5% in length and/or volume) during a surgical intervention on body tissue adjacent to the respective anatomical element. In this example, each of the anatomical elements may be referred to as a rigid anatomical element.

Each of the patient trackers 33a-33d may be associated with a registration between (e.g., pre-operative) patient image data and the patient's body 18.

The patient image data may represent at least a part of the patient's body 18. The patient image data may comprise at least one of two-dimensional image data and three-dimensional image data. For example, the patient image data may comprise computed tomography, CT, image data of at least a part of the patient's body 18. As another example, the patient image data may comprise magnetic resonance, MR, image data of at least a part of the patient's body 18.

The method may comprise associating a registration to each of the patient trackers. The method may comprise obtaining or determining a registration for each of the patient trackers. Such registrations may be used, to transform coordinates from the patient image data into "real-world" coordinates. To this end, a registration associated with a given patient tracker may define a transformation (e.g., defining three translational and three rotational components) between a coordinate system of the patient image data and a "real-world" coordinate system, also referred to as patient tracker coordinate system, having a fixed spatial relationship relative to the given patient tracker.

Various techniques for determining such transformations and registrations are known in the art. For example, positions of points acquired on a surface of the patient's body 18 in relation to a given patient tracker 33a-33d may be matched to positions of the patient's surface as represented by the patient image data to obtain a registration between the given patient tracker and the patient image data.

As the poses of the patient trackers 33a-33d differ from one another, the registrations associated with the patient trackers also differ from one another. In other words, each of the patient trackers 33a-33d may be associated with a registration that is specific for that patient tracker.

In step 204, instrument tracking information indicative of tracked poses of two or more medical instruments is obtained.

The instrument tracking information may be obtained from the tracking unit 6 and/or the robot 10. The instrument tracking information may be determined by the at least one processor 12 based on feedback from the robot 10, as explained above. The instrument tracking information in one example indicates a current tracked pose for each of the two or more medical instruments (e.g., for each of the medical instruments 24, 26, 28).

At least one of the medical instruments for which the instrument tracking information is obtained may be handled by a robot. Alternatively, or in addition, at least one (e.g., two or more) of the medical instruments for which the instrument tracking information is obtained may be (e.g., simultaneously) handled by different persons. In the example of Fig. 1, the medical instrument 28 is handled by the robot 10 and the medical instruments 24, 26 are simultaneously handled by different persons.

The method may further comprise an optional step 206 in which one or more sets are determined. As an alternative to optional step 206, or in addition thereto, the method may comprise obtaining set information indicative of one more sets. That is, one or more sets may be predefined and obtained by the at least one processor 12. Whenever it is referred to a set being "determined" herein, this encompasses the determination as part of the method in step 206 and the alternative of obtaining a set that has already been determined accordingly (e.g., in a step separate from the method).

The method disclosed herein uses not only one set, but a plurality of sets. Each set comprises (i) at least one of the medical instruments (e.g., for which the instrument tracking information is obtained in step 204) and (ii) at least one of the patient trackers (e.g., for which the patient tracking information is obtained in step 202). In the example of Fig. 1, a first set may comprise the medical instrument 24 and the patient tracker 33a, a second set may comprise the medical instruments 26, 28 and the patient tracker 33b, and a third set may comprise the medical instruments 24, 26 and the patient trackers 33c, 33d.

The sets used in the method disclosed herein (e.g., in step 208) differ from one another in the at least one of the patient trackers.

As explained above, each of the patient trackers may be associated with a specific registration, which would result in each of the sets also differing in the registrations. In case a set comprises only one patient tracker, it would be associated with only one registration. In case a set comprises two or more patient trackers, it would be associated with the registrations of these two or more patient trackers.

At least one of the sets may comprise at least one of the patient trackers that is not comprised in any other one of the sets. In the aforementioned example of Fig. 1, the first set comprises only the patient tracker 33a as a patient tracker, the second set only comprises the patient tracker 33b as a patient tracker and the third set only comprises the patient trackers 33c, 33d as patient trackers. In this example, each patient tracker is set-specific. In this example, each set only comprises patient trackers that are not comprised in any other one of the sets.

At least one of the sets may comprise at least one of the patient trackers that is comprised in another one of the sets. That is, one or more of the sets may comprise a non-exclusive or non-set-specific patient tracker.

In a first example, at least one of the sets comprises two or more patient trackers, wherein each of these two or more patient trackers has a fixed relationship to a (e.g., tracker-specific or different) anatomical element of the patient's body 18. That is, a set may comprise multiple trackers that are fixed relative to different anatomical elements. Thus, the multiple trackers of such a set may move relative to one another.

In a second example, each of the at least one of the patient trackers within a same set may have a fixed spatial relationship (e.g., be fixedly arranged and/or have a fixed pose) relative to a common anatomical element of the patient's body. In other words, all patient trackers within the same set may have a fixed spatial relationship relative to the common (e.g., same) anatomical element. Thus, these patient trackers may not move relative to one another. In the example of Fig. 1, the patient tracker 33a of the first set is fixedly attached relative to the vertebra 46, the patient tracker 33b of the second set is fixedly arranged relative to the vertebra 56 and the the patient trackers 33c, 33d of the third set have a fixed position and orientation relative to the femur 62. The registrations of the patient tracker(s) having a fixed spatial relationship relative to the common anatomical element may differ from one another, but may remain constant relative to one another even if anatomical elements of the patient's body 18 move over time.

The common anatomical element may differ from one set to another. In the example of Fig. 1, the common anatomical element of the first set is the vertebra 26, the common anatomical element of the second set is the vertebra 56 and the common anatomical element of the third set is the femur 62.

In one particular example, each of the sets comprises exactly one patient tracker, not two or more patient trackers.

The sets may differ from one another in the at least one of the medical instruments. In the aforementioned example of Fig. 1, the first set may comprise the instrument 24, the second set may comprise the instruments 26, 28 and the third set may comprise the instruments 24, 26. In this case, each of the sets differs from any other set in one or more medical instruments.

At least one of the sets may comprise at least one of the medical instruments that is not comprised in any other one of the sets. Put differently, at least one of the sets may comprise an exclusive or set-specific medical instrument. In the aforementioned example of Fig. 1, the second set comprises the set-specific medical instrument 28, which is not comprised in the first or the third set.

At least one of the sets may comprise at least one of the medical instruments that is comprised in another one of the sets. In other words, at least one of the sets may comprise a non-exclusive of non-set-specific medical instrument. In the aforementioned example of Fig. 1, the first set comprises the non-exclusive medical instrument 24, which is also comprised in the third set, and the second set comprises the non-set-specific medical instrument 26, which is also comprised in the third set.

At least one of the sets may be determined (e.g., in step 206) based on the patient tracking information and the instrument tracking information. The at least one of the sets may be determined based on a spatial relationship between one or more tracked patient trackers 33a-33d and one or more tracked medical instruments 24-28. One may say that medical instruments and patient trackers can be assigned to a same set based on their relative tracked positions and/or orientations.

At least one of the sets may be determined based on a relative pose between (i) the at least one of the medical instruments of that set and (ii) at least one of the patient trackers of that set. At least one of the sets may be determined based on a relative pose between (i) the at least one of the medical instruments of that set and (ii) at least one (e.g., a closest one) of the one or more anatomical elements having the fixed spatial relationship relative to the at least one of the patient trackers of that set. The latter relative pose may be determined based on a pose of the at least one (e.g., the closest one) of the one or more anatomical elements as represented by the patient image data, the registration associated with the at least one of the patient trackers of that set and the tracked pose of the at least one of the medical instruments of that set. In other words, the tracked pose of a medical instrument may be compared with poses of anatomical elements represented by the patient image data using different registrations associated with respective patient trackers having fixed spatial relationships to these anatomical elements, and if such a relative pose fulfils a predefined criterion (e.g., a maximum distance) the respective medical instrument and the respective patient tracker may be grouped together in a set. The at least one of the sets may be determined based on the relative pose indicating that the at least one of the medical instruments is aligned with the at least one of the one or more anatomical elements having the fixed spatial relationship relative to the at least one of the patient trackers. In the example of Fig. 1, the axis 27 of the shaft 25 of the medical instrument 24 points towards the vertebra 46 which means the instrument 24 is aligned with the vertebra 26. The vertebra 26 has a fixed spatial relationship relative to the patient tracker 33a. Thus, in this example, the instrument 24 and the patient tracker 46 are grouped into the first set. The at least one of the sets may be determined based on the relative pose indicating that the at least one of the medical instruments is positioned within a predefined (e.g., spatial) region that is associated with the at least one of the one or more anatomical elements having the fixed spatial relationship relative to the at least one of the patient trackers. In the example of Fig. 1, the tips 29, 31 of the instruments 26, 28 both lie within the spatial region 66 that is associated with the vertebra 56. Thus, in this example, the instruments 26, 28 are both grouped into the second set together with the patient tracker 33b fixedly arranged relative to the vertebra 56.

It is also possible for at least one of the sets to be determined based on a user input. For example, a user may select one or more of a plurality of tracked medical instruments and one or more of a plurality of tracked patient trackers and the selected instrument(s) and patient tracker(s) may then be grouped together in a set. The user input may be obtained by the at least one processor 12 via a user input device such as a touch screen (e.g., of the display unit 8). Other variants for determining a set are also possible. For example, one or more sets may be predefined (e.g., by using (e.g., optical) trackers for the instruments and as the patient trackers that are specific for a given set).

In step 206, based on the patient tracking information and the instrument tracking information, simultaneous display of multiple navigation views is triggered, each of the navigation views being determined based on a respective (e.g., navigation-view-specific) set. That is, the multiple navigation views are based on a plurality of sets, and each navigation view is determined based on exactly one set. Such a set comprises, as explained above, (i) at least one of the medical instruments and (ii) at least one of the patient trackers. The sets differ from one another in the at least one of the patient trackers and, optionally, in the at least one of the medical instruments.

The multiple navigation views are determined based on the patient tracking information and the instrument tracking information and, optionally, based on the registrations associated with the patient trackers of the sets. The method comprises determining the multiple navigation views (e.g., as part of step 208 or between steps 206 and 208) based on the patient tracking information and the instrument tracking information and, optionally, based on the registrations associated with the patient trackers of the sets, and triggering simultaneous display of the determined multiple navigation views. Whenever it is referred to a navigation view being "determined" herein, this encompasses the determination of the navigation view as part of the method and an alternative of obtaining a corresponding navigation view that has already been determined accordingly (e.g., in a step separate from the method).

Two or more of the navigation views may be triggered to be displayed on a common display unit (e.g., the display unit 8 shown in Fig. 1). At least one of the navigation views may be triggered to be individually displayed on another display unit (e.g., separate from the common display unit). In other words, the at least one of the navigation views may be triggered to be displayed on an individual display unit. In all such variants, the multiple navigation views may be triggered to be displayed at the same time, for example for different users in the operating room 22.

Each of the navigation views may be determined for a different one of the sets. Each of the navigation views may be specific for the respective set used to determine that navigation view. Each of the navigation views may be determined based on and/or may be specific for the pose(s) of the medical instrument(s) of the respective set. Each of the navigation views may be determined based on and/or may be specific for the pose(s) of the patient tracker(s) of the respective set.

At least one (e.g., each one) of the navigation views may be determined based on the patient image data and the registration(s) associated with the patient tracker(s) of the respective set used to determine that navigation view.

In case the respective set comprises only one patient tracker, the registration associated with that patient tracker may be used and the at least one navigation view may indicate a relative pose between the at least one instrument of the set and the at least one anatomical element having a fixed spatial relationship relative to the only patient tracker of that set.

In case the respective set comprises two or more patient trackers that are fixedly arranged relative to one another via a common anatomical element (e.g., as described for the second example above), an average of the transformations comprised in the registrations associated with the two or more patient trackers may be used. The registration(s) associated with the patient tracker(s) of the respective set used to determine that navigation view may be used for determining a relative pose between the medical instrument(s) of the respective set and the patient image data. The at least one navigation view may indicate this relative pose between the medical instrument(s) of the respective set and the patient image data.

In case the respective set comprises two or more patient trackers that are fixedly arranged relative to one another via a common anatomical element (e.g., as described for the second example above) or in case the respective set comprises two or more patient trackers that are movably arranged relative to one another (e.g., as described for the first example above), the at least one navigation view may indicate, for a plurality of the patient trackers in the set, a pose of the at least one anatomical element having a fixed relationship relative to the respective patient tracker. The at least one navigation view may indicate a relative pose between (i) the at least one anatomical element having a fixed relationship relative to a first one of the patient trackers in the set and (ii) the at least one anatomical element having a fixed relationship relative to a second one of the patient trackers in the set. The at least one navigation view may indicate an angle and/or distance between (i) (e.g., a first surface such as a vertebral endplate surface of) the at least one anatomical element having a fixed relationship relative to a first one of the patient trackers in the set and (ii) (e.g., a second surface such as a vertebral endplate surface of) the at least one anatomical element having a fixed relationship relative to a second one of the patient trackers in the set.

At least one (e.g., each one) of the navigation views may indicate a relative pose between (i) a medical instrument of the set used to determine the at least one of the navigation views and (ii) a portion of the patient's body 18 as represented in (e.g., pre-operative) patient image data. At least one (e.g., each one) of the navigation views may indicate a relative pose between (i) a medical instrument of the set used to determine the at least one of the navigation views and (ii) at least one of the one or more anatomical elements having the fixed spatial relationship relative to one or more of the at least one of the patient trackers of the same set. The at least one (e.g., each one) of the navigation views may further indicate a pose of another medical instrument of the set or of another set.

A navigation view as disclosed herein may indicate a pose of a medical instrument relative to a portion of the patient's body 18 by indicating the pose of the medical instrument as an overlay on an image of the patient's body 18, the image being generated or extracted from (e.g., pre-operative) patient image data. The image and/or the patient image data may be registered to the patient tracker of the set for which that navigation view is determined. As explained above, such a registration may allow associating positions in the image with tracked positions and vice versa to determine the navigation view(s).

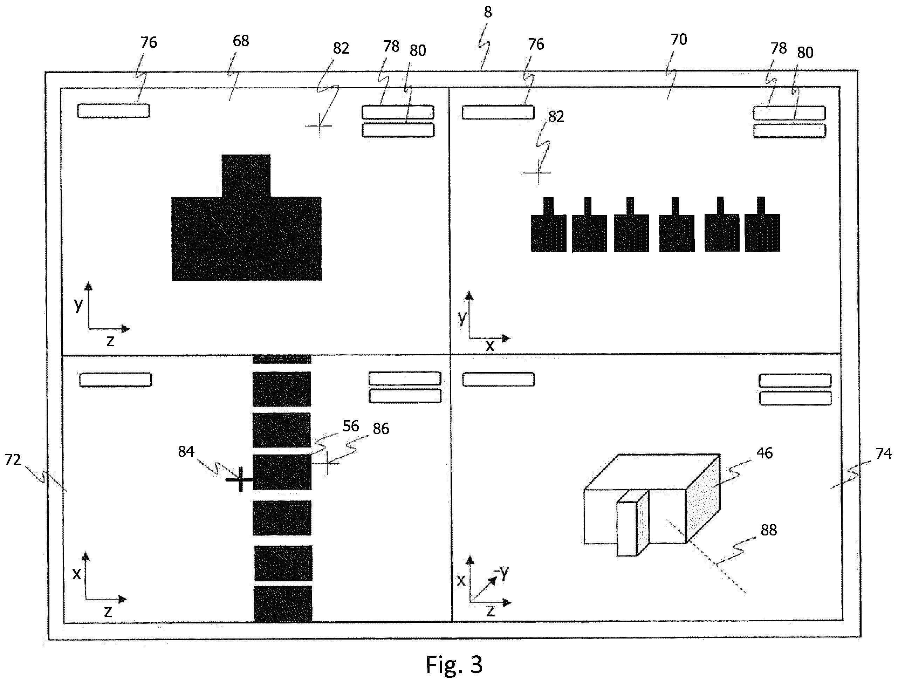

Fig. 3 schematically illustrates exemplary navigation views in accordance with the present disclosure. In this example, four navigation views 68-74 are simultaneously displayed on a common screen of display unit 8, which may be referred to as a split-screen visualization. In this example, each navigation view contains a first selection field 76 indicating a currently selected view type, a second selection field 78 indicating a medical instrument of the set associated with the respective navigation view and a third selection field 80 indicating a vertebra having a fixed spatial relationship relative to a patient tracker 33a-33d of the set associated with the respective navigation view.

A user may select the view type, medical instrument and/or vertebra using these selection fields 76-80, which will result in a display of a correspondingly updated navigation view. Such a change of a set once the navigation views are displayed is indicated with arrow 210 in Fig. 2.

It is also possible for the sets to change automatically in case one or more predefined conditions are fulfilled (e.g., in case a medical instrument moves from one spatial region to another, e.g., from the region 64 into the region 66). In case the patient tracker 33a-33d and/or the medical instrument of the set of the respective navigation view change their pose, the respective navigation view may be updated accordingly to allow for a "live" navigation. This is indicated with arrow 212 in Fig. 2.

In Fig. 3, the x-axis indicates the superior direction, the y-axis indicates the posterior direction and the z-axis indicates the lateral direction. The navigation view 68 is an axial view of the vertebra 46 in which a position of the tip of the instrument 24 is indicated in the form of crosshairs 82. The position of the instrument 24 of the first set relative to the vertebra 46 is determined via the patient tracker 33a of the first set and its associated registration relative to the patient image data, which improves navigational accuracy compared with a case in which for example the tracker 33b is used for navigation the instrument 24 on the vertebra 46. The navigation view 70 is a sagittal view of the spine 60 as represented by the patient image data, in which a position of the tip of the instrument 24 is indicated in the form of crosshairs 82. Again, the position of the instrument 24 of the first set relative to the vertebra 46 is determined via the patient tracker 33a of the first set and its associated patient-to-image registration.

The navigation view 72 is a coronal view of the spine 60 as represented by the patient image data, into which positions of the tips 29, 31 are projected and visualized as distinguishable crosshairs 84, 86 to indicate the current poses of the instruments 26, 28 relative to the vertebra 56. The positions of the tips 29, 31 relative to the vertebra 56 are determined via the patient tracker 33b, namely based on a registration between the patient image data and the patient tracker 33b, thereby improving navigational accuracy compared with a case in which the patient tracker 33a, for example, is used as a reference.

The navigation view 74 is a perspective view of the vertebra 46 as represented by the patient image data, in which the trajectory 27 of the instrument 24 is indicated as a dashed line 88. In this case, the user has opted for the pose of the instrument 24 relative to the patient's body 18 to be determined via both the patient trackers 33c, 33d. That is, the set used for determining the navigation view 74 comprises not one, but two patient trackers 33c, 33d. An average registration can be determined based on a first registration associated with the tracker 33c and a second registration associated with the tracker 33d, and the average registration can be used to transform coordinates from the patient image data into the real-world coordinate system or vice versa. On may also use the trackers 33c, 33d to track a position in the middle between these two trackers, and use a registration relative to this middle position instead of the average registration. Using multiple patient trackers per set may also improve navigational accuracy.

As will be apparent to those skilled in the art, other variants of the navigation views 68-74 are also possible, for example using freely chosen viewing directions, visualizing other portions of the patient's body 18 or visualizing the poses of the instruments in a different manner. The navigation views may visualize further components such as pre-planned implants, points, lines, planes or segmentation boundaries.

Details of the inventive technique will now be phrased in other words to provide a better understanding thereof. Although reference signs of Figs. 1-3 may be used in the following, these are not to be understood as limiting the technique to the specific details shown in these figures.

In certain scenarios, it may be desirable to get navigation feedback from more than just one medical instrument 24-28 and more than one anatomical element of the patient's body 18. Some systems only provide navigation feedback from multiple combinations of medical instruments and anatomical elements in sequence (one after the other and only one at a moment in time), which makes the entire procedure lengthy and it consumes a lot of time until the entire feedback is collected. When using a single patient tracker arranged fixedly relative to an anatomical element, a surgical procedure is often limited to that anatomical element. Namely, when operating on other anatomical elements (e.g., remote vertebrae), their poses in space may change while such changes are not reflected by the anatomical element associated to the tracker. Thus, when operating on a plurality of anatomical elements, a single patient tracker might need to be physically moved from one to the other anatomical element, and a registration for that anatomy might need to be repeated.

The present technique allows using multiple patient trackers and multiple medical instruments. The trackers and instruments are grouped in different sets to cover different possible combinations of patient trackers and medical instruments for feedback. In case two instruments 24, 26 and two patient trackers 33a, 33b are used, one or more of the following navigation views may be triggered to be displayed: 24 relative to 33a, 26 relative to 33a, 24 relative to 33b, 26 relative to 33b. All sets may be tracked and displayed at once, if desired. For example, in a 2 x 2 window layout, all four possible combinations may be displayed simultaneously, and each of the windows may offer different possible viewing options (e.g. sagittal view, coronal view, axial view, instrument-oriented view, 3D view). When using more than one screen for displaying the navigation views, the number of displayed windows and sets can be increased even further. Thus, not only feedback on a single instrument is provided, but navigation views for instruments handled by different surgeons and robots can be visualized simultaneously. Such a simultaneous display may preferable over a long-lasting sequential feedback and cumbersome and distorting switching between navigation views.

The technique disclosed herein may enable multiple users to simultaneously navigate a plurality of medical instruments at a same patient. Each user may use his own navigated instrument and get his real-time feedback of the pose of the navigated instrument used. The users may share a common screen for their individual navigation feedback or each user may have a separate screen for navigation feedback. There may even be more screens than users if wanted. The navigation experience for each user may thus be independent from the other users, which means all users may get the full navigation functionality. For example, the may not be a master-slave relation between instruments handled by different surgeons, were the master instrument determines an image to be used for overlaying instrument poses and the slave instruments are merely projected into that image.

In addition to surgeons, robots can be involved, for example. Navigation feedback on a robot's end effector pose may be provided. Navigation feedback of navigated dilators may be provided. In general, the technique disclosed herein enables navigation of any tracked medical instrument which pose is of interest, independently whether it is a manual handled instrument, a robotic tool or a mechanical fixture.

The pose of each navigated instrument can be displayed appropriately in a respective navigation view, independently from any other instruments. "Appropriate" here means not as a projection in the views of other instruments but in a navigation view dedicated to the respective instrument that may include the navigated tip and the shaft of the instrument as well if wanted. Examples of navigation views include a trajectory view focused on a vertebra Lumbar 1 and used for navigating a Jamshidi needle as medical instrument; a coronal view focused on Lumbar 1 and used for navigating a Microscope; a trajectory view focused on a vertebra Lumbar 4 and used for navigating the robot 10; a three-dimensional view focused on the vertebra Lumbar 4 and used for navigating a Pointer as medical instrument.

Using multiple patient trackers may allow for exact navigation at different portions of the patient's body 18. For example, a surgeon and his assistant may both simultaneously navigate a screw into a pedicle. They may work simultaneously at the same vertebra or each of them at a different vertebra where each vertebra may be tracked individually with a separate patient tracker. The system 2 may comprise has two monitors connected for feedback. Two surgeons may thus be working simultaneously on the patient's body 18, their two medical instruments 24, 26 being navigated on two distinct vertebrae having respective patient trackers associated therewith.

Multiple navigation views can provided simultaneously on one or more screens, wherein any tracked medical instrument can be combined with any tracked patient tracker to display imaging contents depending on the relation between the positions of selected tracker and instrument. For example, a sectional image at the position of a certain pointer tip in a certain tracker's coordinate system can be visualized as a navigation view. In addition to instrument tip positions within tracker coordinate systems, sectional images may be displayed as navigation views that are determined by the shaft/trajectory of a certain instrument within a certain tracker's coordinate system. The potential navigation views cover sets patient trackers and medical instruments, and each navigation view may provide the possibility to choose multiple display options (e.g. axial, coronal, sagittal, trajectory 1, trajectory 2, or perpendicular to tip) for the respective set.

The technique disclosed herein may be modified, for example my omitting optional steps, changing the sequential order of steps 202 and 204, or combining some of the method steps. For example, step 204 may be performed in advance of step 202, or steps 202 and 204 may be performed in unison. In one example, tracking data is obtained which includes both the instrument tracking information and the patient tracking information, and potentially some further information such as a timestamp or the like. Modifications of the hardware example illustrated in Fig. 1 may also be possible. For example, a distributed (e.g., cloud) processing system may be provided that is configured to perform the method disclosed herein. Further modifications and advantages of the technique according to the present disclosure may become apparent to those skilled in the art.

## Claims

1. A computer-implemented method for supporting clinical personnel with multiple navigation views (68-74), the method being performed by at least one processor (12) and not comprising a surgical step, the method comprising:
obtaining (202) patient tracking information indicative of tracked poses of two or more patient trackers (33a-33d), each of the patient trackers (33a-33d) having a fixed spatial relationship relative to one or more anatomical elements (46-62) of a patient's body (18);
obtaining (204) instrument tracking information indicative of tracked poses of two or more medical instruments (24-28);
determining, based on the patient tracking information and the instrument tracking information, multiple navigation views (68-74), wherein each of the navigation views (68-74) is determined based on a respective set comprising (i) at least one of the medical instruments (24-28) and (ii) at least one of the patient trackers (33a-33d), wherein the sets differ from one another in the at least one of the patient trackers (33a-33d); and
triggering (208) simultaneous display of the multiple navigation views (68-74).

2. The computer-implemented method of claim 1, wherein
each of the navigation views (68-74) is specific for the respective set used to determine the respective navigation view (68-74).

3. The computer-implemented method of claim 1 or 2, wherein
each of the at least one of the patient trackers (33a-33d) within a same set has a fixed spatial relationship relative to a common anatomical element (46, 56, 62) of the patient's body (18).

4. The computer-implemented method of claim 3, wherein
the common anatomical element (46, 56, 62) differs from one set to another.

5. The computer-implemented method of any one of claims 1 to 4, wherein
the sets differ from one another in the at least one of the medical instruments (24-28).

6. The computer-implemented method of any one of claims 1 to 5, wherein at least one of the following conditions is fulfilled:
at least one of the sets comprises at least one of the medical instruments (28) that is not comprised in any other one of the sets;
at least one of the sets comprises at least one of the medical instruments (24, 26) that is comprised in another one of the sets.

7. The computer-implemented method of any one of claims 1 to 6, wherein
at least one of the sets is determined based on the patient tracking information and the instrument tracking information.

8. The computer-implemented method of any one of claims 1 to 7, wherein
at least one of the sets is determined based on a relative pose between (i) the at least one of the medical instruments (24-28) and (ii) at least one of the one or more anatomical elements (46-62) having the fixed spatial relationship relative to the at least one of the patient trackers (33a-33d).

9. The computer-implemented method of claim 8, wherein
the at least one of the sets is determined based on the relative pose indicating that at least one of the following conditions is fulfilled:
the at least one of the medical instruments (24) is aligned with the at least one of the one or more anatomical elements (46) having the fixed spatial relationship relative to the at least one of the patient trackers (33a);
the at least one of the medical instruments (26, 28) is positioned within a predefined region (66) that is associated with the at least one of the one or more anatomical elements (56) having the fixed spatial relationship relative to the at least one of the patient trackers (33b).

10. The computer-implemented method of any one of claims 1 to 9, wherein
at least one of the sets is determined based on a user input.

11. The computer-implemented method of any one of claims 1 to 10, wherein
at least one of the navigation views (68-74) indicates a relative pose between (i) a medical instrument (24-28) of the set used to determine the at least one of the navigation views (68-74) and (ii) at least one of the one or more anatomical elements (46, 56, 62) having the fixed spatial relationship relative to one or more of the at least one of the patient trackers (33a-33d) of the same set.

12. The computer-implemented method of claim 11, wherein
the at least one of the navigation views (68-74) further indicates a pose of another medical instrument (24-28) of the set or of another set.

13. The computer-implemented method of any one of claims 1 to 12, wherein at least one of the following conditions is fulfilled:
two or more of the navigation views (68-74) are triggered to be displayed on a common display unit (8);
at least one of the navigation views (68-74) is triggered to be displayed on an individual display unit.

14. The computer-implemented method of any one of claims 1 to 13, wherein one of more of the following conditions is fulfilled:
at least one of the medical instruments (28) is handled by a robot (10);
at least two of the medical instruments (24, 26) are handled by different persons.

15. A system (2) comprising at least one processor (12) configured to:
obtain (202) patient tracking information indicative of tracked poses of two or more patient trackers (33a-33d), each of the patient trackers (33a-33d) having a fixed spatial relationship relative to one or more anatomical elements (46-62) of a patient's body (18);
obtain (204) instrument tracking information indicative of tracked poses of two or more medical instruments (24-28);
determine, based on the patient tracking information and the instrument tracking information, multiple navigation views (68-74), wherein each of the navigation views (68-74) is determined based on a respective set comprising (i) at least one of the medical instruments (24-28) and (ii) at least one of the patient trackers (33a-33d), wherein the sets differ from one another in the at least one of the patient trackers (33a-33d); and
trigger (208) simultaneous display of the multiple navigation views (68-74).

16. The system of claim 15, wherein the at least one processor (12) is further configured to perform the computer-implemented method of any one of claims 2 to 14.

17. The system of claim 15 or 16, further comprising at least one of the following components:
one or more display units (8) configured to simultaneously display the navigation views (68-74);
a tracking unit (6) configured to track at least one entity selected from the patient trackers (33a-33d) and the medical instruments (24-28);
a robot (10) configured to handle at least one of the medical instruments (24-28).

18. A computer program comprising instructions which, when performed by at least one processor (12), configure the at least one processor (12) to:
obtain (202) patient tracking information indicative of tracked poses of two or more patient trackers (33a-33d), each of the patient trackers (33a-33d) having a fixed spatial relationship relative to one or more anatomical elements (46-62) of a patient's body (18);
obtain (204) instrument tracking information indicative of tracked poses of two or more medical instruments (24-28);
determine, based on the patient tracking information and the instrument tracking information, multiple navigation views (68-74), wherein each of the navigation views (68-74) is determined based on a respective set comprising (i) at least one of the medical instruments (24-28) and (ii) at least one of the patient trackers (33a-33d), wherein the sets differ from one another in the at least one of the patient trackers (33a-33d); and
trigger (208) simultaneous display of the multiple navigation views (68-74).

19. A data carrier (14) carrying the computer program of claim 18.

## Patentansprüche

1. Computerimplementiertes Verfahren zur Unterstützung von klinischem Personal mit mehreren Navigationsansichten (68-74), wobei das Verfahren von mindestens einem Prozessor (12) durchgeführt wird und keinen chirurgischen Schritt umfasst, wobei das Verfahren umfasst:
Erhalten (202) von Patientennachverfolgungsinformationen, die nachverfolgte Posen von zwei oder mehr Patiententrackern (33a-33d) angeben, wobei jeder der Patiententracker (33a-33d) eine feste räumliche Beziehung relativ zu einem oder mehreren anatomischen Elementen (46-62) eines Körpers (18) eines Patienten hat;
Erhalten (204) von Instrumentennachverfolgungsinformationen, die nachverfolgte Posen von zwei oder mehr medizinischen Instrumenten (24-28) angeben;
Bestimmen mehrerer Navigationsansichten (68-74) basierend auf den Patientennachverfolgungsinformationen und den Instrumentennachverfolgungsinformationen, wobei jede der Navigationsansichten (68-74) basierend auf einer jeweiligen Menge bestimmt wird, die (i) mindestens eines der medizinischen Instrumente (24-28) und (ii) mindestens einen der Patiententracker (33a-33d) umfasst, wobei sich die Mengen in mindestens einem der Patiententracker (33a-33d) voneinander unterscheiden; und
Auslösen (208) einer gleichzeitigen Anzeige der mehreren Navigationsansichten (68-74).

2. Computerimplementiertes Verfahren nach Anspruch 1, wobei
jede der Navigationsansichten (68-74) spezifisch für die jeweilige Menge ist, die zum Bestimmen der jeweiligen Navigationsansicht (68-74) verwendet wird.

3. Computerimplementiertes Verfahren nach Anspruch 1 oder 2, wobei
jeder der mindestens einen Patiententracker (33a-33d) in einer gleichen Menge eine feste räumliche Beziehung relativ zu einem gemeinsamen anatomischen Element (46, 56, 62) des Körpers (18) des Patienten aufweist.

4. Computerimplementiertes Verfahren nach Anspruch 3, wobei
das gemeinsame anatomische Element (46, 56, 62) sich von einer Menge zur anderen unterscheidet.

5. Computerimplementiertes Verfahren nach einem der Ansprüche 1 bis 4, wobei
sich die Mengen in mindestens einem der medizinischen Instrumente (24-28) voneinander unterscheiden.

6. Computerimplementiertes Verfahren nach einem der Ansprüche 1 bis 5, wobei mindestens eine der folgenden Bedingungen erfüllt ist:
mindestens eine der Mengen umfasst mindestens eines der medizinischen Instrumente (28), das in keiner anderen der Mengen enthalten ist;
mindestens eine der Mengen umfasst mindestens eines der medizinischen Instrumente (24, 26), das in einer anderen der Mengen enthalten ist.

7. Computerimplementiertes Verfahren nach einem der Ansprüche 1 bis 6, wobei
mindestens eine der Mengen basierend auf den Patientennachverfolgungsinformationen und den Instrumentennachverfolgungsinformationen bestimmt wird.

8. Computerimplementiertes Verfahren nach einem der Ansprüche 1 bis 7, wobei
mindestens eine der Mengen basierend auf einer relativen Pose zwischen (i) mindestens einem der medizinischen Instrumente (24-28) und (ii) mindestens einem des einen oder der mehreren anatomischen Elemente (46-62) bestimmt wird, die die feste räumliche Beziehung relativ zu mindestens einem der Patiententracker (33a-33d) aufweisen.

9. Computerimplementiertes Verfahren nach Anspruch 8, wobei
die mindestens eine der Mengen basierend auf der relativen Pose bestimmt wird, die angibt, dass mindestens eine der folgenden Bedingungen erfüllt ist:
das mindestens eine der medizinischen Instrumente (24) mit dem mindestens einen des einen oder der mehreren anatomischen Elemente (46) ausgerichtet ist, die die feste räumliche Beziehung relativ zu dem mindestens einen der Patiententracker (33a) aufweisen;
das mindestens eine der medizinischen Instrumente (26, 28) innerhalb eines vordefinierten Bereichs (66) positioniert ist, der mit mindestens einem des einen oder der mehreren anatomischen Elemente (56) verknüpft ist, die die feste räumliche Beziehung relativ zu mindestens einem der Patiententracker (33b) aufweisen.

10. Computerimplementiertes Verfahren nach einem der Ansprüche 1 bis 9, wobei
mindestens eine der Mengen basierend auf einer Benutzereingabe bestimmt wird.

11. Computerimplementiertes Verfahren nach einem der Ansprüche 1 bis 10, wobei
mindestens eine der Navigationsansichten (68-74) eine relative Pose zwischen (i) einem medizinischen Instrument (24-28) der Menge, die zum Bestimmen der mindestens einen der Navigationsansichten (68-74) verwendet wird, und (ii) mindestens einem des einen oder der mehreren anatomischen Elemente (46, 56, 62) angibt, die die feste räumliche Beziehung relativ zu einem oder mehreren der mindestens einen Patiententracker (33a-33d) der gleichen Menge aufweisen.

12. Computerimplementiertes Verfahren nach Anspruch 11, wobei
die mindestens eine der Navigationsansichten (68-74) ferner eine Pose eines anderen medizinischen Instruments (24-28) der Menge oder einer anderen Menge angibt.

13. Computerimplementiertes Verfahren nach einem der Ansprüche 1 bis 12, wobei mindestens eine der folgenden Bedingungen erfüllt ist:
die Anzeige von zwei oder mehreren der Navigationsansichten (68-74) wird auf einer gemeinsamen Anzeigeeinheit (8) ausgelöst;
die Anzeige mindestens einer der Navigationsansichten (68-74) wird auf einer individuellen Anzeigeeinheit ausgelöst.

14. Computerimplementiertes Verfahren nach einem der Ansprüche 1 bis 13, wobei eine oder mehrere der folgenden Bedingungen erfüllt sind:
mindestens eines der medizinischen Instrumente (28) wird von einem Roboter (10) gehandhabt;
mindestens zwei der medizinischen Instrumente (24, 26) werden von unterschiedlichen Personen gehandhabt.

15. System (2) mit mindestens einem Prozessor (12), das eingerichtet ist zum:
Erhalten (202) von Patientennachverfolgungsinformationen, die nachverfolgte Posen von zwei oder mehr Patiententrackern (33a-33d) angeben, wobei jeder der Patiententracker (33a-33d) eine feste räumliche Beziehung relativ zu einem oder mehreren anatomischen Elementen (46-62) eines Körpers (18) eines Patienten hat;
Erhalten (204) von Instrumentennachverfolgungsinformationen, die nachverfolgte Posen von zwei oder mehr medizinischen Instrumenten (24-28) angeben;
Bestimmen mehrerer Navigationsansichten (68-74) basierend auf den Patientennachverfolgungsinformationen und den Instrumentennachverfolgungsinformationen, wobei jede der Navigationsansichten (68-74) basierend auf einer jeweiligen Menge bestimmt wird, die (i) mindestens eines der medizinischen Instrumente (24-28) und (ii) mindestens einen der Patiententracker (33a-33d) umfasst, wobei sich die Mengen in mindestens einem der Patiententracker (33a-33d) voneinander unterscheiden; und
Auslösen (208) einer gleichzeitigen Anzeige der mehreren Navigationsansichten (68-74).

16. System nach Anspruch 15, wobei der mindestens eine Prozessor (12) weiterhin dazu eingerichtet ist, das computerimplementierte Verfahren nach einem der Ansprüche 2 bis 14 auszuführen.

17. System nach Anspruch 15 oder 16, weiterhin umfassend mindestens eine der folgenden Komponenten:
eine oder mehrere Anzeigeeinheiten (8), die dazu eingerichtet sind, die Navigationsansichten (68-74) gleichzeitig anzuzeigen;
eine Trackingeinheit (6), die dazu eingerichtet ist, mindestens eine Entität nachzuverfolgen, die aus den Patiententrackern (33a-33d) und den medizinischen Instrumenten (24-28) ausgewählt ist;
einen Roboter (10), der dazu eingerichtet ist, mindestens eines der medizinischen Instrumente (24-28) zu handhaben.

18. Computerprogramm, das Anweisungen umfasst, die, wenn sie von mindestens einem Prozessor (12) durchgeführt werden, den mindestens einen Prozessor (12) konfigurieren zum:
Erhalten (202) von Patientennachverfolgungsinformationen, die nachverfolgte Posen von zwei oder mehr Patiententrackern (33a-33d) angeben, wobei jeder der Patiententracker (33a-33d) eine feste räumliche Beziehung relativ zu einem oder mehreren anatomischen Elementen (46-62) eines Körpers (18) eines Patienten hat;
Erhalten (204) von Instrumentennachverfolgungsinformationen, die nachverfolgte Posen von zwei oder mehr medizinischen Instrumenten (24-28) angeben;
Bestimmen mehrerer Navigationsansichten (68-74) basierend auf den Patientennachverfolgungsinformationen und den Instrumentennachverfolgungsinformationen, wobei jede der Navigationsansichten (68-74) basierend auf einer jeweiligen Menge bestimmt wird, die (i) mindestens eines der medizinischen Instrumente (24-28) und (ii) mindestens einen der Patiententracker (33a-33d) umfasst, wobei sich die Mengen in mindestens einem der Patiententracker (33a-33d) voneinander unterscheiden; und
Auslösen (208) einer gleichzeitigen Anzeige der mehreren Navigationsansichten (68-74).

19. Datenträger (14), der das Computerprogramm nach Anspruch 18 trägt.

## Revendications

1. Procédé mis en œuvre par ordinateur destiné à aider le personnel hospitalier à l'aide de vues de navigation multiples (68-74), le procédé étant exécuté par au moins un processeur (12) et ne comprenant pas d'étape chirurgicale, le procédé consistant à:
obtenir (202) des informations de suivi du patient indiquant les positions suivies d'au moins deux dispositifs de suivi de patient (33a-33d), chacun des dispositifs de suivi de patient (33a-33d) ayant une relation spatiale fixe par rapport à un ou plusieurs éléments anatomiques (46-62) du corps du patient (18);
obtenir (204) des informations de suivi d'instrument indiquant les positions suivies d'au moins deux instruments médicaux (24-28);
déterminer, sur la base des informations de suivi du patient et des informations de suivi d'instrument, plusieurs vues de navigation (68-74), chacune des vues de navigation (68-74) étant déterminée sur la base d'un ensemble respectif comprenant (i) au moins un des instruments médicaux (24-28) et (ii) au moins un des dispositifs de suivi de patient (33a-33d), les ensembles différant l'un de l'autre dans au moins un des dispositifs de suivi de patient (33a-33d); et
déclencher (208) l'affichage simultané des vues de navigation multiples (68-74).

2. Procédé mis en œuvre par ordinateur selon la revendication 1, dans lequel
chacune des vues de navigation (68-74) est spécifique à l'ensemble respectif utilisé pour déterminer la vue de navigation respective (68-74).

3. Procédé mis en œuvre par ordinateur selon la revendication 1 ou 2, dans lequel
chacun de l'au moins un parmi les dispositifs de suivi de patient (33a-33d) d'un même ensemble a une relation spatiale fixe par rapport à un élément anatomique commun (46, 56, 62) du corps du patient (18).

4. Procédé mis en œuvre par ordinateur selon la revendication 3, dans lequel
l'élément anatomique commun (46, 56, 62) diffère d'un ensemble à l'autre.

5. Procédé mis en œuvre par ordinateur selon l'une quelconque des revendications 1 à 4, dans lequel
les ensembles diffèrent les uns des autres dans l'au moins un des instruments médicaux (24-28).

6. Procédé mis en œuvre par ordinateur selon l'une quelconque des revendications 1 à 5, dans lequel au moins l'une des conditions suivantes est remplie:
au moins un des ensembles comprend au moins un des instruments médicaux (28) qui n'est pas compris dans l'un quelconque des ensembles;
au moins un des ensembles comprend au moins un des instruments médicaux (24, 26) qui est compris dans un autre des ensembles.

7. Procédé mis en œuvre par ordinateur selon l'une quelconque des
revendications 1 à 6, dans lequel
au moins un des ensembles est déterminé sur la base des informations de suivi du patient et des informations de suivi de l'instrument.

8. Procédé mis en œuvre par ordinateur selon l'une quelconque des revendications 1 à 7, dans lequel
au moins un des ensembles est déterminé sur la base d'une position relative entre (i) l'au moins un des instruments médicaux (24-28) et (ii) au moins un du ou des éléments anatomiques (46-62) ayant une relation spatiale fixe par rapport à l'au moins des dispositifs de suivi de patient (33a-33d).

9. Procédé mis en œuvre par ordinateur selon la revendication 8, dans lequel
l'un au moins des ensembles est déterminé sur la base de la position relative indiquant qu'au moins l'une des conditions suivantes est remplie:
l'un au moins des instruments médicaux (24) est aligné sur l'un au moins parmi le ou les éléments anatomiques (46) ayant une relation spatiale fixe par rapport à l'un au moins un parmi les dispositifs de suivi de patient (33a);
l'au moins un parmi les instruments médicaux (26-28) est positionné dans une zone prédéfinie (66) qui est associée à l'au moins un parmi le ou les éléments anatomiques (56) ayant la relation spatiale fixe par rapport à l'au moins des dispositifs de suivi de patient (33a-33b).

10. Procédé mis en œuvre par ordinateur selon l'une quelconque des revendications 1 à 9, dans lequel
au moins l'un des ensembles est déterminé sur la base d'une entrée d'utilisateur.

11. Procédé mis en œuvre par ordinateur selon l'une quelconque des revendications 1 à 10, dans lequel
l'au moins une des vues de navigation (68-74) indique une position relative entre (i) un instrument médical (24-28) de l'ensemble utilisé pour déterminer l'au moins une des vues de navigation (68-74) et (ii) au moins un parmi le ou les éléments anatomiques (46, 56, 62) ayant une relation spatiale fixe par rapport à un ou plusieurs de l'au moins un parmi les dispositifs de suivi de patient (33a-33d) du même ensemble.

12. Procédé mis en œuvre par ordinateur selon la revendication 11, dans lequel
l'une au moins des vues de navigation (68-74) indique en outre la position d'un autre instrument médical (24-28) de l'ensemble ou d'un autre ensemble.

13. Procédé mis en œuvre par ordinateur selon l'une quelconque des revendications 1 à 12, dans lequel au moins l'une des conditions suivantes est remplie:
au moins deux des vues de navigation (68-74) sont déclenchées pour être affichées sur une unité d'affichage commune (8);
au moins une des vues de navigation (68-74) est déclenchée pour être affichée sur une unité d'affichage individuelle.

14. Procédé mis en œuvre par ordinateur selon l'une quelconque des revendications 1 à 13, dans lequel au moins une ou plusieurs parmi les conditions suivantes est remplie:
au moins un des instruments médicaux (28) est manipulé par un robot (10);
au moins deux des instruments médicaux (24, 26) sont manipulés par des personnes différentes.

15. Système (2) comprenant au moins un processeur (12) configuré pour:
obtenir (202) des informations de suivi du patient indiquant les positions suivies d'au moins deux dispositifs de suivi de patient (33a-33d), chacun des dispositifs de suivi de patient (33a-33d) ayant une relation spatiale fixe par rapport à un ou plusieurs éléments anatomiques (46-62) du corps du patient (18);
obtenir (204) des informations de suivi d'instrument indiquant les positions suivies d'au moins deux instruments médicaux (24-28);
déterminer, sur la base des informations de suivi du patient et des informations de suivi d'instrument, plusieurs vues de navigation (68-74), chacune des vues de navigation (68-74) étant déterminée sur la base d'un ensemble respectif comprenant (i) au moins un des instruments médicaux (24-28) et (ii) au moins un des dispositifs de suivi de patient (33a-33d), les ensembles différant l'un de l'autre dans au moins un des dispositifs de suivi de patient (33a-33d); et
déclencher (208) l'affichage simultané des vues de navigation multiples (68-74).

16. Système selon la revendication 15, dans lequel l'au moins un processeur (12) est en outre configuré pour exécuter le procédé mis en œuvre par ordinateur selon l'une quelconque des revendications 2 à 14.

17. Système selon la revendication 15 ou 16, comprenant en outre au moins l'un des composants suivants:
une ou plusieurs unités d'affichage (8) configurées pour afficher simultanément les vues de navigation (68-74);
une unité de suivi (6) configurée pour suivre au moins une entité choisie parmi les dispositifs de suivi de patient (33a-33d) et les instruments médicaux (24-28);
un robot (10) configuré pour manipuler au moins l'un des instruments médicaux (24, 28).

18. Programme informatique comprenant des instructions qui, lorsqu'elles sont exécutées par au moins un processeur (12), configurent l'au moins un processeur (12) pour:
obtenir (202) des informations de suivi du patient indiquant les positions suivies d'au moins deux dispositifs de suivi de patient (33a-33d), chacun des dispositifs de suivi de patient (33a-33d) ayant une relation spatiale fixe par rapport à un ou plusieurs éléments anatomiques (46-62) du corps du patient (18);
obtenir (204) des informations de suivi d'instrument indiquant les positions suivies d'au moins deux instruments médicaux (24-28);
déterminer, sur la base des informations de suivi du patient et des informations de suivi d'instrument, plusieurs vues de navigation (68-74), chacune des vues de navigation (68-74) étant déterminée sur la base d'un ensemble respectif comprenant (i) au moins un des instruments médicaux (24-28) et (ii) au moins un des dispositifs de suivi de patient (33a-33d), les ensembles différant l'un de l'autre dans au moins un des dispositifs de suivi de patient (33a-33d); et
déclencher (208) l'affichage simultané des vues de navigation multiples (68-74).

19. Support de données (14) portant le programme informatique selon la revendication 18.
